# EUROPEAN PATENT SPECIFICATION

(11) **EP 3 868 347 B1**
(45) Date of publication and mention of the grant of the patent: **29.04.2026**
(21) Application number: 20158649.2
(22) Date of filing: 21.02.2020
(51) Int. Cl.: A61F 13/551

(54) **ABSORBENT ARTICLES HAVING SINGLE-HANDED OPENING AND METHODS**
ABSORBIERENDE ARTIKEL MIT EINHÄNDIGEM ÖFFNEN UND VERFAHREN
ARTICLES ABSORBANTS AYANT UNE OUVERTURE À UNE SEULE MAIN ET PROCÉDÉS

(43) Date of publication of application: 25.08.2021
(73) Proprietor: Ontex BV, 9255 Buggenhout (BE); Ontex Group NV, 9320 Erembodegem (BE)
(72) Inventor: Heege, Thomas, 56761 Düngenheim (DE); Krechel, Andreas, 56759 Kalenborn (DE); Ozgenc, Melis, 9320 Aalst-Erembodegem (BE)
(74) Representative: Saurat, Thibault

(56) References cited:
- EP-A1- 0 597 446
- WO-A1-03/082174
- AU-A1- 2014 227 512
- DE-A1- 102016 106 354

## Description

### TECHNICAL FIELD

The disclosure pertains to the technical field of absorbent hygiene products and methods of making thereof, preferably disposable hygiene products such as tampons.

### BACKGROUND

The disclosure relates to absorbent articles, such as tampons, methods of making, and method of opening, and in particular to improvements for easy handling an opening thereof.

Attempts have been made in the prior art to provide easy and effective opening of packaged devices such as tampons in an intuitive and hygienic way.

Fore example, US20100130954 discloses that tampons are packaged in a substantially cylindrical wrapper formed of a flexible, sheet-like material that is closed with one or more attachment zones having one or more gaps crossed by a line of weakness. The wrapper is closed at both ends and has a longitudinal seam area in which overlapping portions of the flexible, sheet-like material are attached. The longitudinal seam area comprises a substantially longitudinal attachment zone with a first edge and a second edge that defines a seam area. The line of weakness has a first end disposed within the seam area at a distance from a first end of the wrapper package; extends through a gap in the substantially longitudinal attachment zone; continues generally circumferentially about the wrapper; and terminates at a second end disposed within the seam area at the second edge of the substantially longitudinal attachment zone. The line of weakness extends across the seam area, and the wrapper can be removed as a single piece.

Similarly, US20100130953 discloses a packaged elongate intravaginal device that has an overwrap substantially enclosing the device. The overwrap has a longitudinal overlap seam disposed generally parallel to the longitudinal axis. The seam includes one ply of overwrap material disposed at a first margin of a overwrap blank superposed on a second ply of overwrap material disposed at a second margin of the overwrap blank, opposite the first. The overwrap has a substantially continuous line of weakness that intersects a plane including the longitudinal axis of the packaged device at at least three unique locations. The line of weakness extends across the longitudinal overlap seam and includes weakness components superposed in each ply of the overlap seam, and the line of weakness is arranged and configured in a manner to permit the overwrap to remain as a unitary structure upon destruction of the line of weakness.

Document DE 10 2016 106 354 A1 describes a packaged tampon having a cylindrical absorbent body packaged in a single wrapper made of at least one film with has at least one weakening line extending transversely to the longitudinal direction of the tampon with great importance being attached to the fact that it can be opened as quietly and easily as possible.

Whilst the twist-and-open arrangements of the prior art provide an improvement over classical opening systems, they still require two-handed manipulation in order to provide the necessary twist force for opening.

A need therefore exists for absorbent articles, such as tampons, that can be single-handedly opened from the packaging thereof and that make a distinctive sound when opening, and/or tactile impression upon opening so that when a subject single-handedly opens the packaging the subject has an immediate feedback that the tampon can be extracted and used no matter the conditions, e.g. in poor light conditions or complete dark. Moreover, a need exists for such articles that are designed for easy-use by the visually impaired.

### SUMMARY

In a first aspect, the disclosure relates to a packaged elongate intravaginal device having a longitudinal axis and comprising: a. an elongate intravaginal device having a tapered end positioned at the front of the elongate intravaginal device and an oppositely disposed substantially flat end positioned at the rear of the elongate intravaginal device at a distance along the longitudinal axis, wherein the elongate intravaginal device has a diameter **d** and a maximum length **L** extending from said flat end to said tapered end along the longitudinal axis; and b. an overwrap substantially enclosing the elongate intravaginal device and having a longitudinal overlap seam disposed generally parallel to the longitudinal axis and extending along a length of said elongate intravaginal device, wherein: i. the seam includes one ply of overwrap material disposed at a first margin of an overwrap blank superposed on a second ply of overwrap material disposed at a second margin of the overwrap blank, opposite the first margin; and ii. the overwrap has a substantially continuous line of weakness that extends along a circumference of the elongate intravaginal device and across the seam and includes weakness elements superposed in each ply of the seam; wherein the line of weakness originates from two symmetrically disposed ends and wherein the distance between the substantially flat end positioned at the rear of the elongate intravaginal device and the furthest position **P** on the line of weakness along the longitudinal axis is greater than the diameter **d** of the elongate intravaginal device and less than 0.5L, so that said overwrap may be single-handedly opened to expose the rear of the elongate intravaginal device, and wherein the distance between the substantially flat end positioned at the rear of the elongate intravaginal device and the furthest position **P** on the line of weakness along the longitudinal axis is from 1.1**d** to 2**d**.

In a second aspect, the disclosure relates to a method of manufacturing a packaged elongate intravaginal device as described herein, comprising the steps of: (i) unwinding a roll of overwrap material, preferably in the form of a film, typically a liquid impermeable film, having a length extending along a machine direction; (ii) perforating the overwrap material along a line of weakness extending along an axis running substantially parallel, or sinusoidally, to the length of said overwrap material running parallel to the machine direction; (iii) providing an elongate intravaginal device; (iv) cutting the overwrap material along an axis running substantially perpendicular to the line of weakness and/or the length of said overwrap material running parallel to the machine direction, typically to provide a plurality of overwrap blanks having a width extending substantially parallel to the line of weakness and a length substantially perpendicular thereto; (v) wrapping the cut overwrap material around the elongate intravaginal device; and (vi) applying heat at least along a seam of the wrapped elongate intravaginal device to provide a sealed elongate intravaginal device.

In a further aspect, the disclosure relates to a method of opening a packaged elongate intravaginal device comprising the steps of: (i) providing a packaged elongate intravaginal device as described herein; (ii) applying a thumb force such to flex the substantially flat end (3) away from the longitudinal axis (X-X) and so that the line of weakness (8) breaks with a "click" sound, wherein the line of weakness breaks typically abruptly when flexing the substantially flat end away from the longitudinal axis and wherein the overwrap material is a multi-layer film.

Preferably, wherein the thumb is positioned at a position indicated by one or more indicia, and more preferably the flexing applied in a direction indicated by at least one of the indicia.

### BRIEF DESCRIPTION OF THE FIGURES

**Fig. 1** Is a perspective view of a packaged device according to an embodiment of the disclosure.
**Fig. 2** Is a side view of a packaged device according to an embodiment of the disclosure.
**Fig. 3** Is a side view of a packaged device according to an embodiment of the disclosure.
**Fig. 4** Is a planar view of a blank overwrap material according to an embodiment of the disclosure.
**Fig. 5** Is a planar view of a blank overwrap material according to an embodiment of the disclosure.
**Fig. 6** Illustrates the opening of a packaged device according to an embodiment of the disclosure.
**Fig. 7** Illustrates an opened packaged device according to an embodiment of the disclosure.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Turning point or stationary point" as used herein means the point on a curve where the gradient substantially equals to zero. Typically the turning point is positioned at a local maximum of a curve.

The term "overwrap" as used herein refers to a structure, which is formed of a sheet of material and which substantially encloses an individual intravaginal device.

The term "intravaginal device" may mean those devices designed to be placed within the vaginal canal such a tampon, or incontinent/pessary device.

As used herein, the terms "weakness component" and "line of weakness" shall mean a series of weakness elements substantially arranged in a row. These weakness elements may be perforations, areas of reduced thickness, slits, score lines, areas of reduced density, etc. The line of weakness may be perforated mechanically or scraped ultrasonically or with a laser.

Embodiments of the articles and processes according to the disclosure will now be described. It is understood that technical features described in one or more embodiments maybe combined with one or more other embodiments without departing from the intention of the disclosure and without generalization therefrom.

### THE ARTICLE

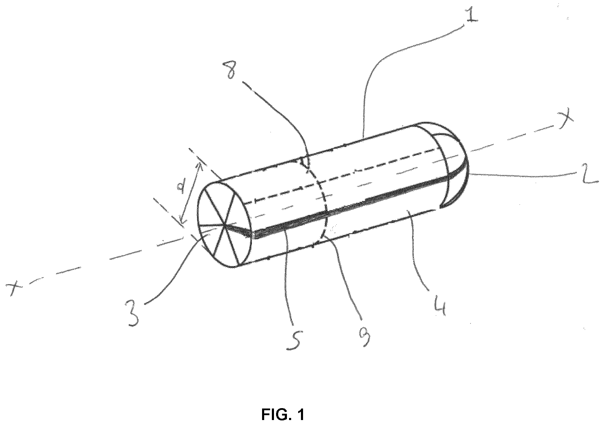

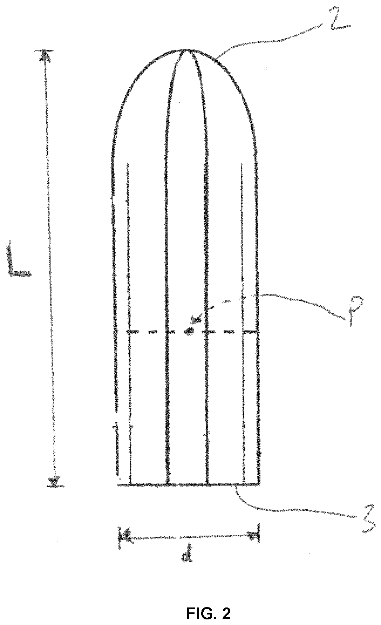

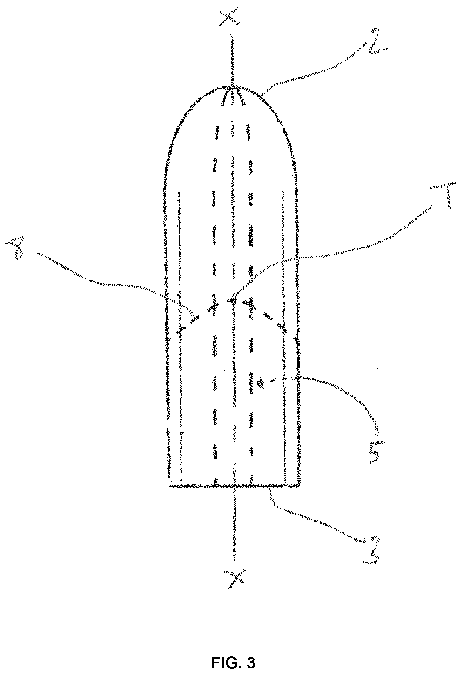

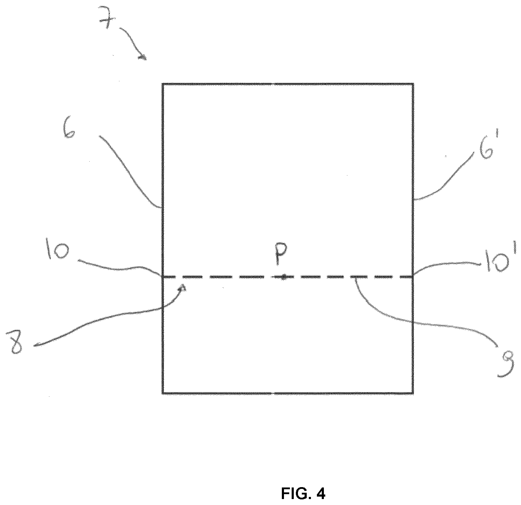

As shown in Figs. 1-7, packaged elongate intravaginal devices herein have a longitudinal axis (X-X) and comprise: a. an elongate intravaginal device (1), preferably a disposable tampon, having a tapered end (2) (also sometimes referred to as the insertion end) positioned at the front of the elongate intravaginal device (1) and an oppositely disposed substantially flat end (3) (herein also referred to as the withdrawal end) positioned at the rear of the elongate intravaginal device (1) at a distance along the longitudinal axis (X-X), wherein the elongate intravaginal device (1) has a diameter **d** and a maximum length L extending from said flat end (3) to said tapered end (2) along the longitudinal axis (X-X). The elongate intravaginal device (1) may comprise a withdrawal string (11) extending from said withdrawal end (3) (and typically joined thereto); and b. an overwrap (4) substantially enclosing the elongate intravaginal device and having a longitudinal overlap seam (5) disposed generally parallel to the longitudinal axis (X-X) and extending along a length of said elongate intravaginal device (1), wherein: i. the seam (5) includes one ply of overwrap material disposed at a first margin (6) of an overwrap blank (7) superposed on a second ply of overwrap material disposed at a second margin (6') of the overwrap blank (7), opposite the first margin (6); and ii. the overwrap has a substantially continuous line of weakness (8) that extends along a circumference of the elongate intravaginal device (1) and across the seam (5) and includes weakness elements (9) superposed in each ply of the seam (5); wherein, the line of weakness (8) originates from two symmetrically disposed ends (10), typically being oppositely disposed on either side of the longitudinal axis (X-X) and generally connected to each other by a common transverse axis that runs perpendicular to the longitudinal axis (X-X), and in that the distance between the substantially flat end (3) positioned at the rear of the elongate intravaginal device (1) and the furthest position P on the line of weakness (8) along the longitudinal axis (X-X) is greater than the diameter d of the elongate intravaginal device (1) and less than 0.5L, so that said overwrap (4) may be single-handedly opened to expose the rear of the elongate intravaginal device (1). It has been found that positioning the opening towards the withdrawal end a better grip may be achieve for single handed opening by use of a thumb, at the same time, depending on the diameter of the intravaginal device, a certain distance from the withdrawal end is needed in order to achieve easy-opening by allowing sufficient displacement of the withdrawal end away from the longitudinal axis when pushing with a thumb. The result is an easy and effective single-handed opening of the overwrap to expose the withdrawal end of the intravaginal device that may further be advantageous to allow hygienically removing the intravaginal device from said overwrap without touching the outer surface thereof that would subsequently be inserted into the vagina.

The distance between the substantially flat end (3) positioned at the rear of the elongate intravaginal device (1) and the furthest position P on the line of weakness (8) along the longitudinal axis (X-X) is from 1.1d to 2d, preferably from 1.2d to 1.9d, even more preferably from 1.3d to 1.8d, even more preferably from 1.4d to 1.7d, wherein d is the diameter of the elongate intravaginal device. Positioning the line of weakness within these ranges provides for optimal single-handed opening as well as sufficient abrupt tearing to promote an audible sound providing signal to the user that the overwrap has opened and the intravaginal device ready to be removed therefrom.

In an embodiment, the line of weakness (8) is arranged and configured in a manner to permit the overwrap to remain as a unitary structure upon destruction of the line of weakness (8). Preferably, the line of weakness (8) is arranged such that a greater force is needed in order to break the seam (5) compared to any other position along said line of weakness (8).

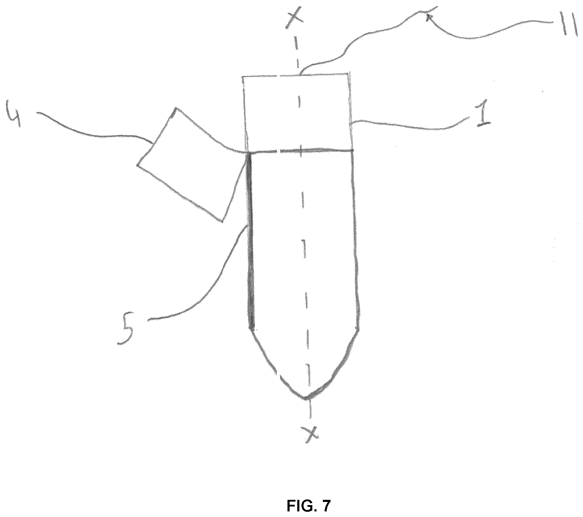

In an embodiment, the line of weakness (8) is arranged such that it does not break, typically does not easily break, at a position of the seam (5). Advantageously this allows the overwrap to hang about the seam (5) upon opening, as illustrated in Fig. 7, and thus remain integral thereto as a unitary structure during extraction of the tampon for more convenient and easy disposal thereafter.

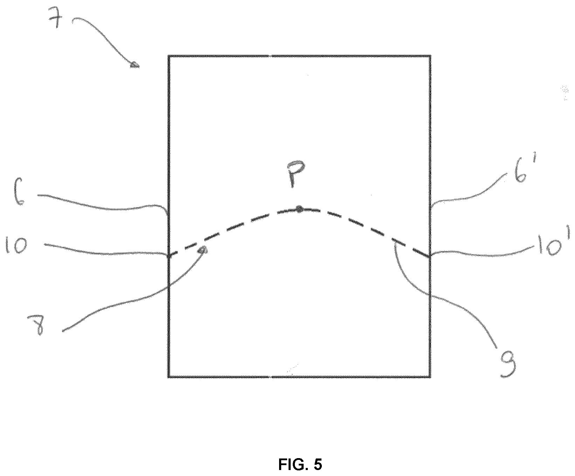

Preferably, the furthest position **P** on the line of weakness is located at a position substantially directly opposite the seam (5). Typically this is achieved by ensuring that said furthest position **P** is positioned substantially at a mid-point of the width of the blank, as illustrated in Fig. 5. Advantageously, this allows to maximise the moment arm length and thus reduce the amount of force needed to open the overwrap with a thumb force as pushing and bending the withdrawal end away from the longitudinal axis X-X.

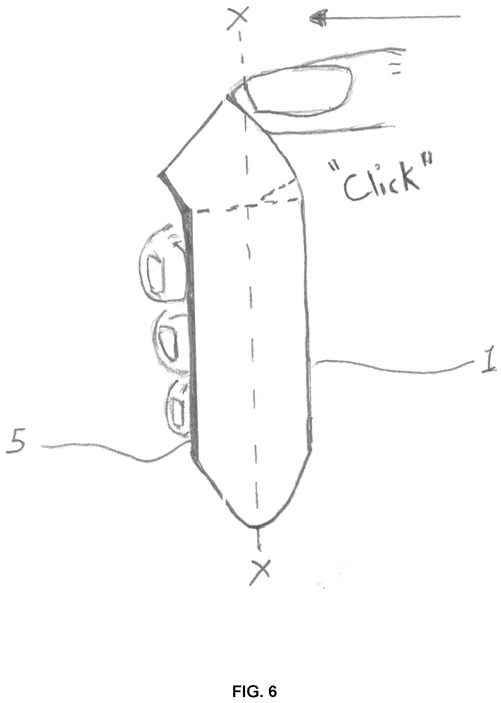

In an embodiment, the seam (5) is provided with a tactile cue that allows the user to easily recognize and grip the wrapped intravaginal device with her fingers positioned over said seam (5). This allows to more intuitively grip the device for single-handed opening by pushing with the thumb that ergonomically at this point would be in the most optimal position for applying a thumb-force, as illustrated in Fig. 6.

In an embodiment, the packaged elongate intravaginal device makes a "click" sound when breaking the line of weakness (8) upon pushing the substantially flat end (3) with a thumb. Advantageously this allows the user to know when the overwrap is open and can be manipulated to remove the intravaginal device from the wrap, this especially useful when in discrete places where visibility and lighting may not be optimal. Moreover, this feature allows for easy use by visually impaired subjects.

Preferably, the line of weakness (8) breaks typically abruptly when flexing the substantially flat end (3) away from the longitudinal axis (X-X). This generally promotes an audible "click" sound to be generated.

In an embodiment, the line of weakness (8) is curved and preferably comprises one, typically a single, turning point (typically corresponding to the furthest position P) positioned opposite the seam (5), preferably wherein the apex thereof corresponds to the position furthest away from the substantially flat end (3). Advantageously, this further reduces the thumb-force required to open the wrap.

In an embodiment, the line of weakness may originate proximate a withdrawal end (generally corresponding to the substantially flat end (9)) of the packaged elongate intravaginal device with a ratio of open length to land length along the line of weakness of about 1:1 and a ratio of open length to land length along the line of weakness distal the withdrawal end of less than about 2:1.

The line of weakness does not necessarily have to be straight. As formed in the overwrap, the weakness elements and resultant line of weakness may be one or more diagonal lines, a curved line or a line that changes direction by e.g. having angles, curves, and/or inflections such as inflection points. Nevertheless, in order to create a distinguishable "click" sound it is preferred to avoid having too many undulations or wavy patterns typically such that there are no more than 5 waves (e.g. peaks and troughs) along the circumference of the elongate intravaginal device. In order to obtain the line of weakness in this type of embodiments, the sheet of material may have the weakness components in the appropriate pattern. In one embodiment, the line of weakness originates in the withdrawal portion of the tampon and inclines such that it terminates in the central portion. In particular, the line of weakness can be inclined at an angle from the plane perpendicular to the longitudinal axis. In another embodiment, the line of weakness originates in the lower 5-25% of the sheet (or blank) and terminates in the upper central area. The angle of inclination is balanced against the desire to maintain the wrapper in a single piece. The smaller the angle, the greater the component of force applied to the package is translated into shear force that is able to rupture the line of weakness. However, a small angle of inclination provides greater opportunity for the wrapper material to tear between the ends of the line of weakness to result in two separated package remnants. The larger the angle, the lesser opportunity for the wrapper material to tear between the ends of the line of weakness to result in two separated package remnants.

In an embodiment, the overwrap material is a multi-layer film, preferably comprising at least three layers and wherein at least one of the layers interposed between other two of said layers comprises a plurality of voids, even more preferably wherein said multi-layer film comprises at least 4 layers, even more preferably 5 layers. An example of such film materials is STD gloss white voided film manufactured and sold by Treofan Germany GmbH & Co. KG, Am Prime Parc 17, 65479 Raunheim / Germany. Advantageously laminate film layers according to the above have been found to provide a very distinguishable and loud "click" sound. Without wishing to be bound by theory, the voids in the inner layers of the laminate act as internal weakening structures and at the same time as sounding board that amplifies the tearing sound upon failure of the material.

In an embodiment, the weakness elements (9) comprise, preferably consist of, a plurality of spaced apart cuts and wherein the pitch between said cuts is from 0.2mm to 2.5mm, preferably from 0.5mm to 2mm, even more preferably from 0.7mm to 1.5mm. By using a specific pitch between weakness elements in areas of the overwrap, it is possible to provide an overwrap that opens completely around the tampon, remains in a single unitary piece but provides for hygienic removal of the tampon from the overwrap.

In an embodiment, the overwrap material has an elongation at break of from 100% to 200%, preferably from 130% to 170%, in the machine direction and an elongation at break of from 25% to 80%, preferably from 30% to 70%, in a transverse direction being perpendicular to the machine direction, according to test method ISO527. Advantageously, this permits a balance between low opening force and sufficiently audible "click" sound when the breakage point is reached.

In one preferred embodiment, the thumb force needed bend the substantially flat end (3) away from the longitudinal axis X-X and to rupture the line of weakness of the overwrap is less than about 25 Newtons ("N"), preferably from 1N to 20N, more preferably from 3N to 15N, even more preferably from 4N to 10N.

In an embodiment, the device comprises indicia positioned on the outermost surface of the overwrap (4) typically at a position opposite the seam (5) corresponding to a position adjacent the furthest position P and/or turning point (T), preferably wherein the indicia is positioned proximal to the turning point or apex of the line of weakness (8), said indicia indicating the position and/or direction of providing a thumb pressure to flex the substantially flat end (3) away from the longitudinal axis (X-X). Preferably, the indicia is in the form of a print having a shape, preferably wherein said shape being selected from one or more arrows, a finger-print, and combinations thereof. Advantageously, this way the user intuitively grasps the packaged device and applies the thumb force at the most optimal position for easy single-handed opening.

### THE METHOD

The disclosure further relates to a method of manufacturing a packaged elongate intravaginal device, comprising the steps of: (i) unwinding a roll of overwrap material, preferably in the form of a film, typically a liquid impermeable film, having a length extending along a machine direction; (ii) perforating the overwrap material along a line of weakness (8) extending along an axis running substantially parallel, or sinusoidally, to the length of said overwrap material running parallel to the machine direction; (iii) providing an elongate intravaginal device (1); (iv) cutting the overwrap material along an axis running substantially perpendicular to the line of weakness (8) and/or the length of said overwrap material running parallel to the machine direction typically to provide a plurality of overwrap blanks having a width extending substantially parallel to the line of weakness (8) and a length substantially perpendicular thereto; (v) wrapping the cut overwrap material around the elongate intravaginal device (1); and (vi) applying heat at least along a seam (5) of the wrapped elongate intravaginal device (1) to provide a sealed elongate intravaginal device.

In an embodiment, step (v) comprises wrapping each overwrap blank around each elongate intravaginal device (1) such that the length of each blank extends along the maximum length L of the elongate intravaginal device (1) and the width of each blank extends along the circumference of said elongate intravaginal device (1). Typically wherein, the width of each said blank is greater than the circumference of said elongate intravaginal device (1), preferably from 2% to 15% greater, more preferably from 5% to 10% greater.

Preferably, the overwrap material comprises a repeating print comprising a plurality of sequentially spaced apart indicia along the machine direction. The indicia may be in the form as described herein above in relating embodiments.

The disclosure herein further relates to a method of opening a packaged elongate intravaginal device comprising the steps of: (i) providing a packaged elongate intravaginal device as described herein above; (ii) applying a thumb force such to flex the substantially flat end (3) away from the longitudinal axis (X-X) and so that the line of weakness (8) abruptly breaks with a "click" sound, wherein the line of weakness breaks typically abruptly when flexing the substantially flat end away from the longitudinal axid, and wherein the overwrap material is a multi-layer film.

Preferably, wherein the thumb is positioned at a position indicated by one or more indicia, and more preferably the flexing applied in a direction indicated by at least one of the indicia.

In an embodiment, step (ii) of the method of opening, comprises the step of exposing the substantially flat end (3) of the elongate intravaginal device (1) such that a withdrawal string connected thereto is also exposed; and the method further comprises the steps of holding the overwrap with one hand at a position proximal to the tapered end (2) and simultaneously pulling the withdrawal string with the other hand to expose the tapered end (2) from the overwrap; and subsequently wherein the elongate intravaginal device (1) is to be inserted into the vagina of a subject whilst holding the withdrawal string, such that the fingers of the subject do not touch the elongate intravaginal device (1) except for a portion of the substantially flat end (3). Advantageously this allows for appropriate hygienic handling of the device.

## Claims

1. A packaged elongate intravaginal device having a longitudinal axis (X-X) comprising:
a. the elongate intravaginal device (1) having a tapered end (2) positioned at the front of the elongate intravaginal device (1) and an oppositely disposed substantially flat end (3) positioned at the rear of the elongate intravaginal device (1) at a distance along the longitudinal axis (X-X), wherein the elongate intravaginal device (1) has a diameter d and a maximum length L extending from said flat end (3) to said tapered end (2) along the longitudinal axis (X-X); and
b. an overwrap (4) substantially enclosing the elongate intravaginal device and having a longitudinal overlap seam (5) disposed generally parallel to the longitudinal axis (X-X) and extending along a length of said elongate intravaginal device (1), wherein:
i. the seam (5) includes one ply of overwrap material disposed at a first margin (6) of an overwrap blank (7) substantially superposed on a second ply of overwrap material disposed at a second margin (6') of the overwrap blank (7), opposite the first margin (6); and
ii. the overwrap has a substantially continuous line of weakness (8) that extends along a circumference of the elongate intravaginal device (1) and across the seam (5) and includes weakness elements (9) superposed in each ply of the seam (5);
**characterized in that,** the line of weakness (8) originates from two symmetrically disposed ends (10, 10') and **in that** the distance between the substantially flat end (3) positioned at the rear of the elongate intravaginal device (1) and the furthest position **P** on the line of weakness (8) along the longitudinal axis (X-X) is greater than the diameter **d** of the elongate intravaginal device (1) and less than 0.5**L**, so that said overwrap (4) may be single-handedly opened to expose the rear of the elongate intravaginal device (1), wherein the distance between the substantially flat end (3) positioned at the rear of the elongate intravaginal device (1) and the furthest position **P** on the line of weakness (8) along the longitudinal axis (X-X) is from 1.1**d** to 2**d**, preferably from 1.2**d** to 1.9**d**, even more preferably from 1.3**d** to 1.8**d**, even more preferably from 1.4**d** to 1.7**d**, wherein **d** is the diameter of the elongate intravaginal device.

2. A packaged elongate intravaginal device according to the preceding Claim wherein packaged elongate intravaginal device makes a "click" sound when breaking the line of weakness (8) upon pushing the substantially flat end (3) with a thumb, wherein the line of weakness (8) breaks typically abruptly when flexing the substantially flat end (3) away from the longitudinal axis (X-X) and wherein the overwrap material is a multi-layer film, preferably comprising at least three layers and wherein at least one of the layers interposed between other two of said layers comprises a plurality of voids, even more preferably wherein said multi-layer film comprises at least 4 layers, even more preferably 5 layers.

3. A packaged elongate intravaginal device according to any of the preceding Claims wherein the line of weakness (8) is curved and preferably comprises at least one, preferably from one to four, typically a single, turning point (T) positioned opposite the seam (5), preferably wherein the apex thereof corresponds to the furthest position **P.**

4. A packaged elongate intravaginal device according to Claim 1 or to Claims 1 and 3 wherein the overwrap material is a multi-layer film, preferably comprising at least three layers and wherein at least one of the layers interposed between other two of said layers comprises a plurality of voids, even more preferably wherein said multi-layer film comprises at least 4 layers, even more preferably 5 layers.

5. A packaged elongate intravaginal device according to any of the preceding Claims wherein the weakness elements (9) comprise, preferably consist of, a plurality of spaced apart cuts and wherein the pitch between said cuts is from 0.2mm to 2.5mm, preferably from 0.5mm to 2mm, even more preferably from 0.7mm to 1.5mm.

6. A packaged elongate intravaginal device according to any of the preceding Claims wherein the overwrap material has an elongation at break of from 100% to 200%, preferably from 130% to 170%, in the machine direction and an elongation at break of from 25% to 80%, preferably from 30% to 70%, in a transverse direction being perpendicular to the machine direction, according to test method ISO527.

7. A packaged elongate intravaginal device according to any of the preceding Claims comprising indicia positioned on the outermost surface of the overwrap (4) at a position opposite the seam (5), preferably wherein the indicia is positioned proximal to the turning point or apex of the line of weakness (8), said indicia indicating the position and/or direction of providing a thumb pressure to flex the substantially flat end (3) away from the longitudinal axis (X-X).

8. A packaged elongate intravaginal device according to Claim 7 wherein the indicia is in the form of a print having a shape, preferably wherein said shape being selected from one or more arrows, a finger-print, and combinations thereof.

9. A method of manufacturing a packaged elongate intravaginal device according to any of the preceding Claims, comprising the steps of:
(i) unwinding a roll of overwrap material, preferably in the form of a film, having a length extending along a machine direction;
(ii) perforating the overwrap material along a line of weakness (8) extending along an axis running substantially parallel, or sinusoidally, to the length of said overwrap material running parallel to the machine direction;
(iii) providing an elongate intravaginal device (1);
(iv) cutting the overwrap material along an axis running substantially perpendicular to the line of weakness (8) and/or the length of said overwrap material running parallel to the machine direction;
(v) wrapping the cut overwrap material around the elongate intravaginal device (1); and
(vi) applying heat to the wrapped elongate intravaginal device (1) at least along a seam (5) to provide a sealed elongate intravaginal device.

10. A method according to Claim 9 wherein the overwrap material comprises a repeating print comprising a plurality of sequentially spaced apart, and preferably distinct, indicia along the machine direction.

11. A method of opening a packaged elongate intravaginal device comprising the steps of:
(i) providing a packaged elongate intravaginal device according to Claims 1 to 8;
(ii) applying a thumb force such to flex the substantially flat end (3) away from the longitudinal axis (X-X) and so that the line of weakness (8) breaks with a "click" sound, wherein the line of weakness (8) breaks typically abruptly when flexing the substantially flat end (3) away from the longitudinal axis (X-X) and wherein the overwrap material is a multi-layer film, preferably comprising at least three layers and wherein at least one of the layers interposed between other two of said layers comprises a plurality of voids, even more preferably wherein said multi-layer film comprises at least 4 layers, even more preferably 5 layers.

12. A method according to Claim 11 wherein the thumb is positioned at a position indicated by one or more indicia, preferably according to Claim 8, and preferably the flexing applied in a direction indicated by at least one of the indicia.

13. A method according to Claims 11 to 12 wherein step (ii) comprises the step of exposing the substantially flat end (3) of the elongate intravaginal device (1) such that a withdrawal string (11) connected thereto is also exposed; and the method further comprises the steps of holding the overwrap with one hand at a position proximal to the tapered end (2) and simultaneously pulling the withdrawal string with the other hand to expose the tapered end (2) from the overwrap; and subsequently wherein the elongate intravaginal device (1) is to be inserted into the vagina of a subject whilst holding the withdrawal string, such that the fingers of the subject do not touch the elongate intravaginal device (1) except for a portion of the substantially flat end (3).

## Patentansprüche

1. Verpackte längliche intravaginale Vorrichtung mit einer Längsachse (X-X), umfassend:
a. die längliche intravaginale Vorrichtung (1) mit einem verjüngten Ende (2), das an der Vorderseite der länglichen intravaginalen Vorrichtung (1) positioniert ist, und einem gegenüberliegend angeordneten, im Wesentlichen flachen Ende (3), das an der Rückseite der länglichen intravaginalen Vorrichtung (1) in einem Abstand entlang der Längsachse (X-X) positioniert ist, wobei die längliche intravaginale Vorrichtung (1) einen Durchmesser **d** und eine maximale Länge **L** aufweist, die sich von dem flachen Ende (3) zu dem verjüngten Ende (2) entlang der Längsachse (X-X) erstreckt; und
b. eine Umhüllung (4), die die längliche intravaginale Vorrichtung im Wesentlichen umschließt und eine längliche Überlappungsnaht (5) aufweist, die im Allgemeinen parallel zur Längsachse (X-X) angeordnet ist und sich entlang einer Länge der länglichen intravaginalen Vorrichtung (1) erstreckt, wobei:
i. die Naht (5) eine Lage Umhüllungsmaterial einschließt, die an einem ersten Rand (6) eines Umhüllungszuschnitts (7) angeordnet ist, der im Wesentlichen eine zweite Lage Umhüllungsmaterial überlagert, die an einem zweiten Rand (6') des Umhüllungszuschnitts (7), gegenüber dem ersten Rand (6), angeordnet ist; und
ii. die Umhüllung eine im Wesentlichen durchgehende Schwächungslinie (8) aufweist, die sich entlang eines Umfangs der länglichen intravaginalen Vorrichtung (1) und über die Naht (5) erstreckt und Schwächungselemente (9) aufweist, die in jeder Lage der Naht (5) übereinander liegen;
**dadurch gekennzeichnet, dass** die Schwächungslinie (8) von zwei symmetrisch angeordneten Enden (10, 10') ausgeht und dass der Abstand zwischen dem im Wesentlichen flachen Ende (3), das sich an der Rückseite der länglichen intravaginalen Vorrichtung (1) befindet, und der am weitesten entfernten Position **P** auf der Schwächungslinie (8) entlang der Längsachse (X-X) größer als der Durchmesser **d** der länglichen intravaginalen Vorrichtung (1) und kleiner als 0,5**L** ist, sodass die Umhüllung (4) mit einer Hand geöffnet werden kann, um die Rückseite der länglichen intravaginalen Vorrichtung (1) freizulegen, wobei der Abstand zwischen dem im Wesentlichen flachen Ende (3), das an der Rückseite der länglichen intravaginalen Vorrichtung (1) positioniert ist, und der am weitesten entfernten Position **P** auf der Schwächungslinie (8) entlang der Längsachse (X-X) von 1,1**d** bis 2**d**, vorzugsweise von 1,2**d** bis 1,9**d**, noch bevorzugter von 1,3**d** bis 1,8**d**, noch bevorzugter von 1,4**d** bis 1,7**d** beträgt, wobei **d** der Durchmesser der länglichen intravaginalen Vorrichtung ist.

2. Verpackte längliche intravaginale Vorrichtung nach dem vorstehenden Anspruch, wobei die verpackte längliche intravaginale Vorrichtung ein "Klick"-Geräusch erzeugt, wenn die Schwächungslinie (8) beim Drücken des im Wesentlichen flachen Endes (3) mit einem Daumen unterbrochen wird, wobei die Schwächungslinie (8) üblicherweise abrupt unterbrochen wird, wenn das im Wesentlichen flache Ende (3) von der Längsachse (X-X) weg gebogen wird, und wobei das Umhüllungsmaterial ein mehrschichtiger Film ist, vorzugsweise mindestens drei Schichten umfasst und wobei mindestens eine der Schichten, die zwischen zwei anderen der Schichten angeordnet ist, eine Vielzahl von Hohlräumen umfasst, noch bevorzugter, wobei der mehrschichtige Film mindestens 4 Schichten, noch bevorzugter 5 Schichten umfasst.

3. Verpackte längliche intravaginale Vorrichtung nach einem der vorstehenden Ansprüche, wobei die Schwächungslinie (8) gekrümmt ist und vorzugsweise mindestens einen, vorzugsweise einen bis vier, üblicherweise einen einzigen, Wendepunkt (T) umfasst, der gegenüber der Naht (5) angeordnet ist, wobei der Scheitelpunkt davon vorzugsweise der am weitesten entfernten Position **P** entspricht.

4. Verpackte längliche intravaginale Vorrichtung nach Anspruch 1 oder nach den Ansprüchen 1 und 3, wobei das Umhüllungsmaterial ein mehrschichtiger Film ist, der vorzugsweise mindestens drei Schichten umfasst und wobei mindestens eine der Schichten, die zwischen zwei anderen dieser Schichten eingefügt sind, eine Vielzahl von Hohlräumen umfasst, noch bevorzugter, wobei der mehrschichtige Film mindestens 4 Schichten, noch bevorzugter 5 Schichten umfasst.

5. Verpackte längliche intravaginale Vorrichtung nach einem der vorstehenden Ansprüche, wobei die Schwächungselemente (9) eine Vielzahl von beabstandeten Einschnitten umfassen, vorzugsweise daraus bestehen, und wobei der Abstand zwischen den Einschnitten 0,2 mm bis 2,5 mm, vorzugsweise 0,5 mm bis 2 mm, noch bevorzugter 0,7 mm bis 1,5 mm beträgt.

6. Verpackte längliche intravaginale Vorrichtung nach einem der vorstehenden Ansprüche, wobei das Umhüllungsmaterial eine Bruchdehnung von 100 % bis 200 %, vorzugsweise von 130 % bis 170 %, in der Maschinenrichtung und eine Bruchdehnung von 25 % bis 80 %, vorzugsweise von 30 % bis 70 %, in einer transversalen Richtung, die senkrecht zur Maschinenrichtung verläuft, nach dem Prüfverfahren ISO527 aufweist.

7. Verpackte längliche intravaginale Vorrichtung nach einem der vorstehenden Ansprüche, umfassend Angaben, die auf der äußersten Oberfläche der Umhüllung (4) an einer Position gegenüber der Naht (5) positioniert sind, wobei die Angaben vorzugsweise proximal zum Wendepunkt oder zum Scheitelpunkt der Schwächungslinie (8) positioniert sind, wobei die Angaben die Position und/oder die Richtung angeben, in der ein Daumendruck bereitgestellt wird, um das im Wesentlichen flache Ende (3) von der Längsachse (X-X) wegzubiegen.

8. Verpackte längliche intravaginale Vorrichtung nach Anspruch 7, wobei die Angaben in Form eines Abdrucks mit einer Form vorliegen, wobei die Form vorzugsweise aus einem oder mehreren Pfeilen, einem Fingerabdruck und Kombinationen davon ausgewählt ist.

9. Verfahren zur Herstellung einer verpackten länglichen intravaginalen Vorrichtung nach einem der vorstehenden Ansprüche, umfassend die folgenden Schritte:
(i) Abwickeln einer Rolle von Umhüllungsmaterial, vorzugsweise in Form eines Films, das eine Länge aufweist, die sich entlang einer Maschinenrichtung erstreckt;
(ii) Perforieren des Umhüllungsmaterials entlang einer Schwächungslinie (8), die sich entlang einer Achse erstreckt, die im Wesentlichen parallel oder sinusförmig zu der Länge des Umhüllungsmaterials verläuft, die parallel zur Maschinenrichtung verläuft;
(iii) Bereitstellen einer länglichen intravaginalen Vorrichtung (1);
(iv) Schneiden des Umhüllungsmaterials entlang einer Achse, die im Wesentlichen senkrecht zur Schwächungslinie (8) und/oder der Länge des Umhüllungsmaterials verläuft, die parallel zur Maschinenrichtung verläuft;
(v) Umhüllen der länglichen intravaginalen Vorrichtung (1) mit dem zugeschnittenen Umhüllungsmaterial; und
(vi) Anwenden von Wärme auf die eingewickelte längliche intravaginale Vorrichtung (1) mindestens entlang einer Naht (5), um eine abgedichtete längliche intravaginale Vorrichtung bereitzustellen.

10. Verfahren nach Anspruch 9, wobei das Umhüllungsmaterial einen sich wiederholenden Aufdruck umfasst, der eine Vielzahl von sequentiell beabstandeten und vorzugsweise unterschiedlichen Angaben entlang der Maschinenrichtung umfasst.

11. Verfahren zum Öffnen einer verpackten länglichen intravaginalen Vorrichtung, umfassend die folgenden Schritte:
(i) Bereitstellen einer verpackten länglichen intravaginalen Vorrichtung nach einem der Ansprüche 1 bis 8;
(ii) Anwenden einer Daumenkraft, um das im Wesentlichen flache Ende (3) von der Längsachse (X-X) weg zu biegen und so, dass die Schwächungslinie (8) mit einem "Klick"-Geräusch bricht, wobei die Schwächungslinie (8) üblicherweise abrupt bricht, wenn das im Wesentlichen flache Ende (3) von der Längsachse (X-X) weg gebogen wird, und wobei das Umhüllungsmaterial ein mehrschichtiger Film ist, vorzugsweise mindestens drei Schichten umfasst und wobei mindestens eine der Schichten, die zwischen zwei anderen dieser Schichten angeordnet ist, eine Vielzahl von Hohlräumen umfasst, noch bevorzugter, wobei der mehrschichtige Film mindestens 4 Schichten, noch bevorzugter 5 Schichten umfasst.

12. Verfahren nach Anspruch 11, wobei der Daumen in einer Position positioniert wird, die durch eine oder mehrere Angaben, vorzugsweise nach Anspruch 8, angegeben ist, und vorzugsweise die Beugung in einer Richtung erfolgt, die durch mindestens eine der Angaben angegeben ist.

13. Verfahren nach Anspruch 11 bis 12, wobei Schritt (ii) den Schritt umfasst, das im Wesentlichen flache Ende (3) der länglichen intravaginalen Vorrichtung (1) freizulegen, sodass ein damit verbundener Rückholfaden (11) ebenfalls freigelegt wird; und das Verfahren ferner die Schritte umfasst, die Umhüllung mit einer Hand an einer Position in der Nähe des verjüngten Endes (2) zu halten und gleichzeitig mit der anderen Hand an dem Rückholfaden zu ziehen, um das verjüngte Ende (2) von der Umhüllung freizulegen; und anschließend, wobei die längliche intravaginale Vorrichtung (1) in die Vagina eines Subjekts eingeführt werden soll, während der Rückholfaden gehalten wird, sodass die Finger des Subjekts die längliche intravaginale Vorrichtung (1) mit Ausnahme eines Abschnitts des im Wesentlichen flachen Endes (3) nicht berühren.

## Revendications

1. Dispositif intravaginal allongé emballé ayant un axe longitudinal (X-X) comprenant :
a. le dispositif intravaginal allongé (1) ayant une extrémité conique (2) positionnée à l'avant du dispositif intravaginal allongé (1) et une extrémité sensiblement plate (3) disposée de manière opposée positionnée à l'arrière du dispositif intravaginal allongé (1) à une certaine distance le long de l'axe longitudinal (X-X), dans lequel le dispositif intravaginal allongé (1) a un diamètre **d** et une longueur maximale **L** s'étendant de ladite extrémité plate (3) à ladite extrémité conique (2) le long de l'axe longitudinal (X-X) ; et
b. un suremballage (4) enfermant sensiblement le dispositif intravaginal allongé et ayant une jonction de chevauchement (5) longitudinale disposée généralement parallèlement à l'axe longitudinal (X-X) et s'étendant le long d'une longueur dudit dispositif intravaginal allongé (1), dans lequel :
i. la jonction (5) comporte un pli de matériau de suremballage disposé au niveau d'un premier bord (6) d'une ébauche de suremballage (7) sensiblement superposé sur un second pli de matériau de suremballage disposé au niveau d'un second bord (6') de l'ébauche de suremballage (7), à l'opposé du premier bord (6) ; et
ii. le suremballage a une ligne de faiblesse (8) sensiblement continue qui s'étend le long d'une circonférence du dispositif intravaginal allongé (1) et à travers la jonction (5) et comporte des éléments de faiblesse (9) superposés dans chaque pli de la jonction (5) ;
**caractérisé en ce que** la ligne de faiblesse (8) provient de deux extrémités symétriquement disposées (10, 10') **et en ce que** la distance entre l'extrémité sensiblement plate (3) positionnée à l'arrière du dispositif intravaginal allongé (1) et la position la plus éloignée **P** sur la ligne de faiblesse (8) le long de l'axe longitudinal (X-X) est supérieure au diamètre **d** du dispositif intravaginal allongé (1) et inférieure à 0,5 **L,** de sorte que ledit suremballage (4) peut être ouvert d'une seule main pour exposer l'arrière du dispositif intravaginal allongé (1), dans lequel la distance entre l'extrémité sensiblement plate (3) positionnée à l'arrière du dispositif intravaginal allongé (1) et la position la plus éloignée **P** sur la ligne de faiblesse (8) le long de l'axe longitudinal (X-X) va de 1,1 **d** à 2 **d,** de préférence de 1,2 **d** à 1,9 **d,** encore plus préférablement de 1,3 **d** à 1,8 **d,** encore plus préférablement de 1,4 **d** à 1,7 **d,** dans lequel **d** représente le diamètre du dispositif intravaginal allongé.

2. Dispositif intravaginal allongé emballé selon la revendication précédente, dans lequel le dispositif intravaginal allongé emballé émet un son de type « clic » lorsque la ligne de faiblesse (8) est brisée en poussant l'extrémité sensiblement plate (3) avec un pouce, dans lequel la ligne de faiblesse (8) se brise généralement brusquement lorsque l'extrémité sensiblement plate (3) est fléchie à l'écart de l'axe longitudinal (X-X), et dans lequel le matériau de suremballage est un film multicouche, comprenant de préférence au moins trois couches et dans lequel au moins l'une des couches interposées entre deux autres desdites couches comprend une pluralité de vides, encore plus préférablement dans lequel ledit film multicouche comprend au moins 4 couches, encore plus préférablement 5 couches.

3. Dispositif intravaginal allongé emballé selon l'une quelconque des revendications précédentes, dans lequel la ligne de faiblesse (8) est incurvée et comprend de préférence au moins un, de préférence de un à quatre, typiquement un seul, point de retournement (T) positionné à l'opposé de la jonction (5), de préférence dans lequel le sommet de celle-ci correspond à la position la plus éloignée **P.**

4. Dispositif intravaginal allongé emballé selon la revendication 1 ou les revendications 1 et 3, dans lequel le matériau de suremballage est un film multicouche, comprenant de préférence au moins trois couches et dans lequel au moins l'une des couches interposées entre deux autres desdites couches comprend une pluralité de vides, encore plus préférablement dans lequel ledit film multicouche comprend au moins 4 couches, encore plus préférablement 5 couches.

5. Dispositif intravaginal allongé emballé selon l'une quelconque des revendications précédentes, dans lequel les éléments de faiblesse (9) comprennent une pluralité d'entailles espacées, de préférence sont constitués de celles-ci, et dans lequel le pas entre lesdites entailles va de 0,2 mm à 2,5 mm, de préférence de 0,5 mm à 2 mm, encore plus préférablement de 0,7 mm à 1,5 mm.

6. Dispositif intravaginal allongé emballé selon l'une quelconque des revendications précédentes, dans lequel le matériau de suremballage présente un allongement à la rupture allant de 100 % à 200 %, de préférence de 130 % à 170 %, dans le sens machine et un allongement à la rupture allant de 25 % à 80 %, de préférence de 30 % à 70 %, dans une direction transversale perpendiculaire au sens machine, selon la méthode d'essai ISO 527.

7. Dispositif intravaginal allongé emballé selon l'une quelconque des revendications précédentes, comprenant une indication positionnée sur la surface la plus externe du suremballage (4) au niveau d'une position opposée à la jonction (5), de préférence dans lequel l'indication est positionnée à proximité du point de retournement ou du sommet de la ligne de faiblesse (8), ladite indication indiquant la position et/ou la direction de la fourniture d'une pression du pouce pour fléchir l'extrémité sensiblement plate (3) à l'écart de l'axe longitudinal (X-X).

8. Dispositif intravaginal allongé emballé selon la revendication 7, dans lequel l'indication est sous la forme d'une impression ayant une forme, de préférence dans lequel ladite forme est choisie parmi une ou plusieurs flèches, une empreinte digitale et des combinaisons de celles-ci.

9. Procédé pour la fabrication d'un dispositif intravaginal allongé emballé selon l'une quelconque des revendications précédentes, comprenant les étapes consistant à :
i) dérouler un rouleau de matériau de suremballage, de préférence sous la forme d'un film, dont la longueur s'étend le long d'un sens machine ;
(ii) perforer le matériau de suremballage le long d'une ligne de faiblesse (8) s'étendant le long d'un axe sensiblement parallèle, ou sinusoïdal, à la longueur dudit matériau de suremballage s'étendant parallèlement au sens machine ;
(iii) fournir un dispositif intravaginal allongé (1) ;
(iv) couper le matériau de suremballage le long d'un axe sensiblement perpendiculaire à la ligne de faiblesse (8) et/ou à la longueur dudit matériau de suremballage s'étendant parallèlement au sens machine ;
(v) enrouler le matériau de suremballage coupé autour du dispositif intravaginal allongé (1) ; et
(vi) appliquer de la chaleur au dispositif intravaginal allongé (1) enveloppé au moins le long d'une jonction (5) pour fournir un dispositif intravaginal allongé scellé.

10. Procédé selon la revendication 9, dans lequel le matériau de suremballage comprend une impression répétitive comprenant une pluralité d'indications espacées de manière séquentielle, et de préférence distinctes, le long du sens machine.

11. Procédé pour l'ouverture d'un dispositif intravaginal allongé emballé comprenant les étapes consistant à :
(i) fournir un dispositif intravaginal allongé emballé selon les revendications 1 à 8 ;
(ii) appliquer une force du pouce de manière à fléchir l'extrémité sensiblement plate (3) à l'écart de l'axe longitudinal (X-X) et de sorte que la ligne de faiblesse (8) se brise avec un son de type « clic », dans lequel la ligne de faiblesse (8) se brise généralement brusquement lors de la flexion de l'extrémité sensiblement plate (3) à l'écart de l'axe longitudinal (X-X) et dans lequel le matériau de suremballage est un film multicouche, comprenant de préférence au moins trois couches et dans lequel au moins l'une des couches interposées entre deux autres desdites couches comprend une pluralité de vides, encore plus préférablement dans lequel ledit film multicouche comprend au moins 4 couches, encore plus préférablement 5 couches.

12. Procédé selon la revendication 11, dans lequel le pouce est positionné au niveau d'une position indiquée par une ou plusieurs indications, de préférence selon la revendication 8, et de préférence la flexion est appliquée dans une direction indiquée par au moins l'une des indications.

13. Procédé selon les revendications 11 à 12, dans lequel l'étape (ii) comprend l'étape consistant à exposer l'extrémité sensiblement plate (3) du dispositif intravaginal allongé (1) de telle sorte qu'une ficelle de retrait (11) reliée à celui-ci est également exposée ; et le procédé comprend en outre les étapes consistant à maintenir le suremballage d'une main au niveau d'une position proximale à l'extrémité conique (2) et à tirer simultanément la ficelle de retrait de l'autre main pour exposer l'extrémité conique (2) hors du suremballage ; et ensuite dans lequel le dispositif intravaginal allongé (1) doit être inséré dans le vagin d'un sujet tout en maintenant la ficelle de retrait, de telle sorte que les doigts du sujet ne touchent pas le dispositif intravaginal allongé (1) à l'exception d'une partie de l'extrémité sensiblement plate (3).
